**Europäisches Patentamt**

**(19) European Patent Office**

**Office européen des brevets**

(11) Publication number : **0 575 360 B1**

## (12) EUROPEAN PATENT SPECIFICATION

(45) Date of publication of patent specification :
**10.08.94 Bulletin 94/32**

(51) Int. Cl.$^5$ : **B01D 61/02**

(21) Application number : **92905017.7**

(22) Date of filing : **19.02.92**

(86) International application number :
**PCT/EP92/00341**

(87) International publication number :
**WO 92/14539 03.09.92 Gazette 92/23**

(54) **PROCESS OF CONCENTRATION AND PURIFICATION OF ORGANIC COMPOUNDS.**

(30) Priority : **26.02.91 IT MI910493**

(43) Date of publication of application :
**29.12.93 Bulletin 93/52**

(45) Publication of the grant of the patent :
**10.08.94 Bulletin 94/32**

(84) Designated Contracting States :
**AT BE CH DE DK ES FR GB IT LI NL SE**

(56) References cited :
**EP-A- 0 026 281**
**EP-A- 0 391 621**
**GB-A- 1 472 050**
**US-A- 3 836 457**
**US-B- 524 806**
**PATENT ABSTRACTS OF JAPAN vol. 10, no. 245 (C-368)(2301) 22 August 1986 & JP,A,61 078 404 (NITTO ELECTRIC IND. CO. LTD.) 22 APRIL 1986**

(56) References cited :
**DESALINATION vol. 77, no. 1/3, March 1990, AMSTERDAM, NL, pp. 73-84; R. RAUTENBACH et al.: "SEPARATION POTENTIAL OF NANOFILTRATION MEMBRANES"**

(73) Proprietor : **BRACCO S.p.A.**
**Via E. Folli, 50**
**I-20134 Milano (IT)**
Proprietor : **TECNOFARMACI S.p.A.**
**Piazza Indipendenza, 24**
**I-00040 Pomezia (Rome) (IT)**

(72) Inventor : **VISCARDI, Carlo**
**Via E. Folli, 50**
**I-20134 Milano (IT)**
Inventor : **PIVA, Rodolfo**
**Via E. Folli, 50**
**I-20134 Milano (IT)**

(74) Representative : **Minoja, Fabrizio**
**Studio Consulenza Brevettuale,**
**Via Rossini, 8**
**I-20122 Milano (IT)**

EP 0 575 360 B1

Jouve, 18, rue Saint-Denis, 75001 PARIS

## Description

This invention relates to a process aimed at concentrating and purifying raw aqueous solutions of water-soluble non-ionic organic compounds, preferably those which are useful as contrast enhancing agents in diagnostic procedures, such as for example X-ray, NMR and ultrasound diagnosis, through the use of techniques of tangential filtration on membranes. This process also allows the recovery of valuable reactants in excess and of reaction solvents, if any. It is particularly useful for purifying expensive non-ionic iodinated compounds, which now have been widely used in X-ray imaging.

The introduction in X-ray diagnosis of contrast media containing non-ionic iodinated compounds as opacifying agents represented a remarkable progress in the state of the technique, so far that, these media will eventually substitute the traditional iodinated ionic products (see: Grainger and Dawson, Clinical Radiology, 1990, 42, 1-5).

However the synthetic processes and, particularly, the final purification of these products are more complex and expensive than those previously used to obtain the ionic contrast media. In fact, neutral iodinated opacifying agents differ from the ionic ones because they cannot be isolated and purified by precipitation from water due to their high solubility in this solvent. Hence, the following problems must still be conveniently solved: the removal of ionic species, usually inorganic salts, situated in the final reaction mixture, the recovery of valuable reagents in excess and of the water-soluble reaction media. A preferred technique to be performed (see patents DE-A-1 909 439, GB-A-1 472 050, EP-A-0 026 281) is the one based on the submission of the raw solutions of the contrast media to a complex series of operations such as:

- preliminary removal of the solvent, usually dimethylacetamide (DMAC) or dimethylformamide (DMF), by evaporation,
- dilution by water of the residue,
- extraction of the residual reaction medium, preferably with a chlorinated solvent,
- elution of the aqueous phase on a system of columns of cationic and anionic ion-exchange resins,
- concentration of the eluate by evaporation,
- crystallization of the crude residue from hydroalcoholic mixtures in order to remove the last impurities of neutral type.

The drawbacks related to this type of process are clear. For instance, large purification plants for ion-exchange resins are needed and their running is extremely complex and expensive. In addition, a large quantity of thermal energy is required for the concentration of the considerable volumes of water to be used. It is also foreseen the use, the recovery and the removal of polluting and toxic organic solvents such as the chlorinated ones. Last but not least, the concentration of extremely diluted solutions causes the corresponding concentration of impurity traces and the submission of the final product to a long-lasting thermal treatment.

Another process which can be carried out (see patents: EP-A-0 083 964, WO-A-8 908 101) implies the purification of raw solutions of non-ionic contrast media through preparative liquid chromatography. This technique is extremely complex and expensive too, and in addition it is unsuitable for a generalized industrial application.

**EP -A- 0 391 621** generically mentions the possibility to use ultrafiltration or column chromatography as an optional further purification step in the preparation of iodized hydroxyethyl starch.

A general treatise of ultrafiltration and reverse osmosis techniques is found in Handbook of Separation Process Technology, R.W. Rousseau ed., John Wiley and Sons, 1987.

The diafiltration process is described for example in Separation Techniques for Chemical Engineers, P.A. Schweitzer ed., McGraw Hill Book Company, 1988.

The process of this invention performs this technique on highly concentrated solutions of water-soluble neutral iodinated contrast agents, by obtaining extremely favourable and absolutely unexpected results if compared to the common knowledge.

It is known that organic impurities with low relative mass and/or inorganic salts can be removed from aqueous diluted solutions through tangential filtration processes by using ultrafiltration (U.F.) or nanofiltration (N.F.) membranes (Bungay P.M., Lansdale H.K., De Pinho M.N., "Synthetic Membranes: Science, Engineering and Applications", D. Reidel, C 181, 1986 and Applegate L.E., "Membrane Separation Process", Chem. Eng., pages 63-89, 11.06.1984). As these membranes are partially permeable to substances which have relative masses below a given value, part of impurities with lower relative mass permeates together with water, allowing, beyond the concentration of the desired product, a partial purification too. Higher levels of purification can be achieved by performing, subsequently to the concentration stage, a dilution stage with water of the non-permeated solution (retentate). This stage, named diafiltration, involves the make-up/dilution of the retentate with de-ionized water. The diluted retentate is filtered again through the filtrating membrane in order to allow the permeation of residual impurities.

The main drawback of this technique is that, in order to obtain a high level of purity, large amounts of make-up water have to be added to the retentate, i.e. high dilutions are required. That involves considerable equipment size, as a large membrane surface is needed. In addition, if the recovery of some permeated species is needed, and this is the case, the permeate has to be thermally concentrated with heavy energy consumptions and thermal stress.

The use of large solvent volumes brings about also a consequent loss of valuable product. The rejections of the membranes, available on the market, to products with relative mass lower than 2000 (such as non-ionic iodinated contrast media) are unsufficiently high, so that the final yield of the purification process is lower than the one obtained through known methods. This flaw is particularly serious because the cost of the product to be purified is very high and yield reductions cannot be accepted.

Another limiting factor of the process is given by the unfeasibility of the separation, if DP, which is the osmotic pressure difference across the membrane (DP corresponds to the difference of osmotic pressure between the retentate and the permeate) exceeds the maximum working pressure of the membrane module, which usually ranges between 2.5 and 4 MPa. On the other hand, it is known (from Bungay P.M., "Synthetic Membranes", cited ref., pages 110-112) that useful permeate flow rates can be obtained only when the working pressure overcomes DP by 1 or 2 MPa. For this reason, solutions recording a DP transmembrane pressure higher than 2.5 MPa are not conveniently purified by nano- or ultrafiltration. In this respect, Table 1 shows the foreseeable situation, considering the cited literature, for a raw solution containing for example, the (L)-5-(2-hydroxy-propionyl)amino-2,4, 6-triiodo-bis-(1,3-dihydroxy-isopropyl)isophtalamide [IOPAMIDOL, Bracco's patent GB-A-1 472 050 Example 1, compound A].

**TABLE 1:** Foreseeable osmotic and transmembrane pressures for aqueous solutions containing IOPAMIDOL, sodium chloride (NaCl), 2-amino-1,3-propandiol (APD), dimethylacetamide (DMAC), operating at 20°C.

| Components | Foreseen rejections a) -- | Solution A concentrations (Mol/l) | Solution B concentrations (Mol/l) |
|---|---|---|---|
| Iopamidol | 0.99 | 0.70 | 0.20 |
| NaCl | 0.20 | 2.10 | 0.60 |
| APD | 0.40 | 1.68 | 0.48 |
| DMAC | 0.30 | 4.58 | 1.31 |
| Total osmotic pressure (MPa) b) | | 26.8 | 7.6 |
| ΔΠ transmembrane pressure (MPa) c) | | 8.6 | 2.4 |

a) rejection values for each component have been derived from the data supplied by manufacturer of the membrane type which was used (NANOFILM[TM] NF40, FilmTec Corporation/Dow Chemical), or experimentally from aqueous solutions of the pure components.

b) the osmotic pressure of the solution to be

purified has been calculated according to Van't Hoff's equation (Bungay P.M., "Synthetic Membranes", cited ref., pages 109-153).

c) the foreseeable transmembrane pressure has been calculated by difference between the osmotic pressure of the retentate and of the permeate.

When using a solution of Iopamidol 0.7M (Solution A), a very high osmotic pressure and a definitely unfavourable DP transmembrane pressure are foreseen, while the maximum concentration of the same compound, useful in principle for an acceptable purification, is foreseen at 0.2 Mol/l (Solution B). This concentration limit is too low and will eventually have a negative impact on volumes of liquid to be used and consequently on the equipment sizes and the economic running of the same.

One would have expected that ultrafiltration or nanofiltration applied to non-ionic iodinated contrast media solutions would be either impossible, due to extremely high pressure, or uncompetitive in comparison with the known methods when worked with diluted solutions.

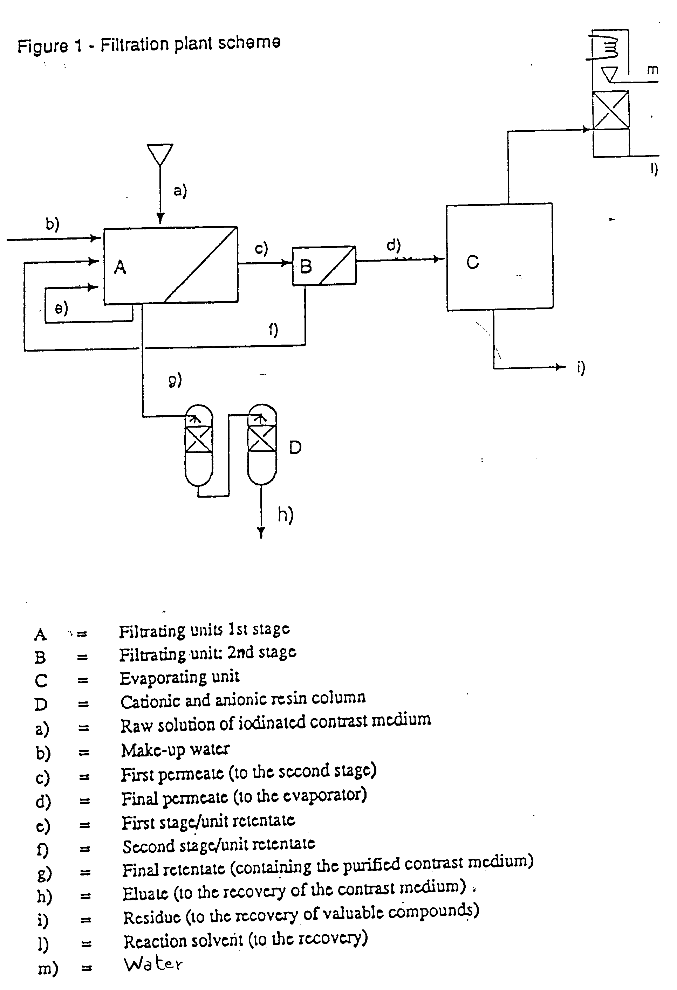

On the contrary, the process of this invention recorded excellent and fully unexpected degrees of purification and recovery of the desired products, thanks to the realization of an innovative filtrating unit which can be run in a non-traditional way, that's to say, by using highly concentrated solutions of the contrast agent and limited water volumes for the make-up of the retentate while keeping the system working pressure within the usual values for the operation. The filtration apparatus has been built by connecting two filtrating groups in series, as shown in the enclosed Figure 1. The second group is analogous to the first one, but has a smaller size and has been designed to recover the small amounts of iodinated compound which remain in the permeate from the first stage, without losses of final product. The ratio between the membrane surfaces of the two filtrating units (unit one /unit two) can range from 1.5 to 6, preferably from 2.5 to 4. Suitable membranes can be sorted out among the commercial ones featuring a rejection to sodium chloride (measured at concentrations of 2000 ppm, pressure of 1.6 MPa, temperature of 25°C) not higher than 85%, preferably lower than 70%, and rejection to raffinose higher than 85%, preferably higher than 95%. By way of example, thin-layer membranes can be cited and particularly: TFM™ G-5 (Desal), NANOFILM™ NF40 (FilmTec/Dow Chemical), ROMEM-BRA™ SU-200S, SU-220S, SU-600 (Toray), NTR 7410, NTR 4550 (Nitto), MOLSEP™ DRA-4020 (Daicel), OS-MOTICS OSMO 411TM012, 411TMX02, 411TBQ01, or asymmetrical polysulphone membranes i.e., PSRO (Millipore). Of course, if the skilled technician uses other types of membranes with similar features, the results are immediate, so, the above mentioned list is absolutely non-limiting for this invention. Tangential filtrating units can be tubular or preferably equipped with flat or spiral wound membranes.

Working temperatures range from 10°C to 90°C according to the membrane used, preferably from 25°C to 45°C.

Working pressures range from 1.5 to 5.5 MPa, preferably from 2 to 3.5 MPa.

The filtrating unit is connected to an evaporation-condenser group to recover the solvents, if any, and to concentrate, before their recovery, salts, valuable reactants in excess and reaction by-products, which are in the permeate. In addition, it can be integrated in the latter phase, with a guard of small-volume ion-exchange resins, providing the full elimination of last traces of ionic species, if still present. The running of these columns does not involve substantial energy consumption or considerable extra-costs, due to their limited size.

This filtrating unit can be surprisingly run under conditions of extremely high concentrations of the contrast agent. In fact, aqueous solutions to be treated usually contain: non-ionic water-soluble iodinated compounds at concentrations ranging between 15 and 60% in weight (w/w), preferably between 20 and 50%, inorganic salts, such as chlorides, bromides, iodides, sulphites of alkaline or alkaline-earth metals or of ammonium or alkylammonium with a relative mass lower than about 150, organic compounds, generally aminoalcohols with a relative mass lower than about 200, water-soluble solvents, such as for instance dimethylacetamide, dimethylformamide, ethanol, dimethylaminoethanol in concentrations not higher than 25% (w/w), preferably not higher than 15%.

The raw solution containing contrast agent, inorganic salts, organic compounds at a relative mass lower than about 200 and solvents (DMAC, DMF and so on) is pumped on the membranes of the first filtrating group. Water, salts, organic compounds at relative mass lower than 200 and solvents, if any, permeate through the membranes and the retentate, partially concentrated and purified as to contrast agent, is recycled at the first stage after dilution with a little amount of de-ionized make-up water. The permeate, which still contains small amounts of the iodinated compound, proceeds towards the second filtrating group. From here, the new retentate is streamed back to the first filtrating group in order to fully purify the recovered contrast medium, while the permeate of the second filtrating group consists of an aqueous solution of salts, organic compounds to be recovered, solvents, if any, and is contrast-agent free.

This solution is concentrated by evaporation and submitted to the recovery of valuable species in it.

The make-up water, used during the diafiltration stage, does not usually exceed 12 kg per mole of the iodinated compound, preferably is less or equal to 8 kg/mol.

The degree of purification obtained by this process is such that the total amount of residual impurities in the final recycled retentate does not exceed 10% of the initial one, preferably 5%. The filtration parameters, in particular the solution and dilution water feed rates, as well as the retentates flow rates and, of course, the operative pressures, are to be experimentally chosen in correlation with the filtration membranes size and porosity in order to obtain the above mentioned purity degrees.

The final recycled retentate can be percolated, if necessary, on the small guard of columns of ionexchange resins to remove any traces of inorganic salts before being submitted to crystallization of the final product.

According to the process of this invention the purification levels obtained as a function of the amount of make-up water fed during the diafiltration step, are surprisingly better than the foreseeable ones, as reported in Table 2.

**TABLE 2** Percentage residue levels of impurities in an aqueous solution containing Iopamidol, NaCl, APD, DMAC, referred to the amount of the make-up water added per mole of Iopamidol

| Make-up water (l) per mole of Iopamidol | Percentage residue impurities | | | | | a) |
|---|---|---|---|---|---|---|
| | NaCl (rejection 0.2) | | APD (rejection 0.4) | | DMAC (rejection 0.3) | b) |
| | c) | d) | c) | d) | c) | d) |
| 0 | 100 | 100 | 100 | 100 | 100 | 100 |
| 6 | 38.3 | 2.1 | 48.7 | 9.0 | 43.2 | 3.5 |
| 12 | 14.7 | 0.1 | 23.7 | 0.6 | 18.6 | 0.3 |

a) calculated by considering equal to 100 the amounts of initial impurity.

b) rejection values for each component have been derived from the data supplied by the membrane manufacturer of NANOFILM$^{TM}$ NF40, or experimentally by aqueous solutions of the pure component.

c) foreseeable values according to the equation (Aptel P., Clifton M., "Synthetic Membranes", cited ref., pages 249-305):

$$C_A = C^\circ_A \cdot exp \ [-Vw/V^\circ (1-R_A)]$$

where:

$V_w$ = make-up water volume

$V°$ = retentate volume

$C_A$ = concentration of component A

$C°_A$ = initial concentration of component A

$R_A$ = membrane rejection to component A.

d) experimental values.

The process of this invention allows, for the first time, the concentration and the purificaton of raw solutions of X-ray water-soluble non-ionic opacifying agents in a simple, economic and environmentally acceptable way.

In particular, it allows higher recovery yields of the contrast compound and the other valuable components, the elimination of large amounts of basic and acid reactants needed for regenerating the ionexchange resins, beyond their total removal, and a remarkable reduction of the steam necessary for the concentration of the eluates. Finally, it avoids extracting the reaction medium with organic toxic solvents such as chlorinated solvents. All this implies considerable operating advantages from the economical and environmental point of view.

Table 3 shows the advantages that the process of this invention brings to the state of the art for the purification of 1000 kg of Iopamidol.

**TABLE 3** Total consumption of the process of desalination of 1000 kg of Iopamidol through tangential filtration and recovery of valuable by-products. Comparison with the state of the art.

| | Process according to the invention | Process according to the state of the art |
| --- | --- | --- |
| | kg | kg |
| Steam | 9,500 | 16.500 |
| De-ionized water | 10,000 | 83,000 |
| HCl 32% | 720 | 4,000 |
| NaOH 30% | 664 | 4,400 |
| Water cooling | 220,000 | 360,000 |

The process of this invention can be also applied to solutions of any non-ionic water-soluble contrast agents, either for use in X-ray or for use in NMR diagnosis.

In particular it can be preferably applied to the solutions of any non-ionic water-soluble iodinated compounds, even if they are monomers, dimers o trimers.

The following experimental examples describe the advantages of this invention without limiting it. The further possible changes of the indicated parameters are absolutely clear to the skilled technician.

## EXAMPLE 1

Purification of (L)-5-(2-hydroxy-propionyl)amino-2,4,6-triiodo-bis-(1,3-dihydroxy-isopropyl)-isophtalamide (Iopamidol: patent GB-A-1 472 050, Example 1).

70 kg of an aqueous solution containing 15.5 kg of Iopamidol (22%, w/w), 2.3 kg of sodium chloride, 1.6 kg of sodium acetate, 4.4 kg of 2-amino-1,3-propandiol and 12 kg of dimethylacetamide are fed in the tank of the two-stage filtrating plant, which is equipped with a total of 4m of a TFM™ DESAL-G-5 membrane (one 2.5″ x 40″ spiral module for the first stage, and one 2.5″ x 21″ spiral module for the 2nd stage). In a first step (1.5 h), the solution is concentrated up to about 37 kg, by operating at 30°C at a working pressure of 3 MPa and at a recirculation rate of 750 l/h for both stages. Then, the diafiltration stage begins by continually reintegrating the water through a dosing pump. The process is stopped after 6.5 h when the conductivity falls under 1000 mS/cm and the final retentate, containing 99.6% of the initial Iopamidol (HPLC determination), is percolated on a couple of columns of ion-exchange resins and subsequently crystallized. 14.6 kg of pure product, complying with the requested analytical features (purification yield: 94.2%), are obtained.

The permeate, weighting approximately 120 kg, contains less than 0.4% of the initial Iopamidol and more than 96% of the other initial species in the mixture. The permeate gets through the recovery stage of valuable components. The recovery is quite simple and cheap thanks to the relatively small amount of diluting water.

## EXAMPLE 2

Purification of 5-(N-methyl-hydroxyacetyl)amino-2,4,6-triiodo-bis-(2,3-dihydroxy-propyl)-isophtalamide (Iomeprol: EP-A-0 026 281, Example 11).

50 kg of an aqueous solution containing 14.2 kg of Iomeprol (28%, w/w), 1.9 kg of sodium chloride, 1.5 kg of sodium acetate, 0.9 kg of 1-amino-2,3-propandiol and 15 kg of dimethylaminoethanol are fed in the tank of the filtrating unit equipped with 4 m² of NANOFILM™NF40 membrane in spiral wound modules, as described in Example 1. The purification is carried out in the same way as in Example 1. 13.2 kg of pure product are obtained (overall purification yield: 93%).

## EXAMPLE 3

Purification of 1,3-bis-[3-(L-2-hydroxy-propionyl)amino-5-(1,3-dihydroxy-isopropyl)aminocarbonyl-2,4,6-triiodo-benzoyl-amino]-2-hydroxypropane (Italian Patent Application n° 22088 A/90, Example 1).

40 kg of an aqueous solution containing 14.8 kg of the above mentioned product (37% w/w), 1.1. kg of sodium chloride, 2.4 kg of sodium acetate and 0.5 kg of 1,3-diamino-2-hydroxypropane are purified with the procedure described in Example 1. 14.4 kg of pure product are obtained (purification yield: 97.3%).

**TABLE 4**   Average   percentage   composition   of   fluids described in Figure 1.

| Components | Fluids (% composition in weight) | | | | | | |
|---|---|---|---|---|---|---|---|
| | a) | d) | g) | h) | i) | l) | m) |
| Contrast agent | 25 | traces | 50 | 50 | traces | - | - |
| Inorganic salts | 5 | 4 | traces | - | 25 | - | - |
| Exceeding reactives | 7 | 4 | 1 | traces | 50 | - | - |
| Organic solvent | 13 | 7 | - | - | - | 50 | 1 |
| $H_2O$ | 50 | 85 | 49 | 50 | 25 | 50 | 99 |

## Claims

1.  A process for the concentration and purification of non-ionic water-soluble, iodinated contrast enhancing organic compounds from their aqueous solutions, wherein their concentration ranges from 15 to 60% by weight, and which contain, as dissolved impurities, inorganic salts and/or water-soluble residual unconsumed organic reactants at a relative mass lower than about 200 and/or water-soluble organic solvents, said process comprising the following steps:

a) feeding said aqueous solution into a first filtration stage of a two-stage, cascade connected, tangential filtration apparatus, said apparatus having filtration membranes, said membranes having a rejection to sodium chloride not higher than 85% and rejection to raffinose higher than 85%;

b) passing the filtrate/permeate from said first stage to said second filtration stage, the ratio of said filtrating membrane surfaces between said first and said second stage ranges from 1.5 to 6;

c) continuously recycling the retentate from said second filtration stage to the retentate from said first filtration stage, diluting the resulting retentate with water, the amount of said water not exceeding 12 kg per mole of said organic compound being subject to the purification;

d) repeating b) to c) on said resulting retentate to obtain a degree of purification of said organic compound in the final retentate from said first stage such that the total amount of residual impurities does not exceed 10% of the initial one.

2. The method according to claim 1, characterized in that step d) is repeated to reach the total amount of residual impurities not exceeding 5% of the initial one.

3. A process according to claims 1-2, characterized in that the ratio of said filtrating membrane surfaces between said first and said second stage ranges from 2.5 to 4.

4. The process according to claims 1-3, characterized in that said membranes have a rejection to sodium chloride not higher than 70% and rejection to raffinose higher than 95%.

5. A process according to anyone of the preceding claims, wherein the working temperature of said first and second stage ranges from 10° to 90°C.

6. A process according to anyone of the preceding claims, wherein the working temperature of said first and second stage ranges from 25° to 45°C.

7. A process according to anyone of the preceding claims, wherein the working pressure of said first and second stage ranges from 1.5 to 5.5 MPa.

8. A process according to anyone of the preceding claims, wherein the working pressure of said first and second stage ranges from 2 to 3.5 MPa.

9. A process according to claim 1, wherein said filtration apparatus is connected to an evaporation-condenser group to recover volatile components from the permeate from said second stage.

10. A process according to claims 1-9, wherein said final retentate is passed through a guard of anionic and cationic ion-exchange resins.

11. A process according to claim 1, wherein in step c) said amount of water does not exceed 8 kg per mole of said organic compound.

12. A process according to claim 1, wherein the non-ionic water-soluble, iodinated contrast enhancing organic compound is the X-ray opacifying agent (L)-5-(2-hydroxy-propionyl)amino-2,4,6-triiodo-bis-(1,3-dihydroxy-isopropyl)isophtalamide.

13. A process according to claim 1, wherein the non-ionic water-soluble, iodinated contrast enhancing organic compound is the X-ray opacifying agent 5-(N-methyl-hydroxyacetyl)amino-2,4,6-triiodo-bis-(2,3-dihydroxy-propyl)-isophtalamide.

14. A process according to claim 1, wherein the non-ionic water-soluble, iodinated contrast enhancing organic compound is the X-ray opacifying agent 1,3-bis-[3-(L-2-hydroxy-propionyl)amino-5-(1,3-dihydroxy-isopropyl)aminocarbonyl-2,4,6-triiodo-benzoyl-amino]1-2-hydroxypropane.

15. A two-stage tangential filtration plant to perform the process of claims 1-14, said plant comprising two filtrating stages (A) and (B) connected in series the ratio of the filtrating membrane surfaces between said first stage (A) and said second stage (B) ranging from 1.5 to 6, an inlet for feeding said first stage (A) with raw solution of iodinated organic compound, characterized in that:

- the retentate outlet of said first stage (A) is connected by means of a recycling circuit e) to the same first stage (A);

- the <u>permeate</u> outlet of said first stage (A) is connected to the feed inlet of said second stage (B) by means of the circuit c);
- the <u>retentate</u> outlet of said second stage (B) is connected by means of a recycling circuit f) to said first stage (A);
- said first stage (A) is provided with a connection for the make-up water line b);
- the <u>permeate</u> outlet of said second stage (B) goes to waste or to a secondary recovery system through the circuit d);
- the final <u>retentate</u> outlet of said first stage (A) goes to the main recovery circuit g) for the recovery of the pure iodinated compound.

16. A filtration plant according to claim 15 wherein the <u>permeate</u> outlet of said second stage (B) is connected through the circuit d) to an evaporating unit c, said evaporating unit being equipped with a condensing unit.

17. A filtration plant according to claim 15 wherein the final <u>retentate</u> outlet of said first stage (A) is connected through the circuit g) to a guard of anionic and cationic resins.


**Patentansprüche**

1. Verfahren zum Konzentrieren und Reinigen von nicht-ionischen, wasserlöslichen, jodierten kontrastverbessernden organischen Verbindungen aus ihren wäßrigen Lösungen, in denen sie in einer Konzentration in dem Bereich von 15 bis 60 Gew.-% vorliegen, und die in gelöster Form Verunreinigungen, anorganische Salze und/oder wasserlösliche restliche unverbrauchte organische Reaktanten mit einer relativen Masse von weniger als etwa 200 und/oder wasserlösliche organische Lösungsmittel enthalten, wobei das Verfahren die folgenden Stufen umfaßt:
   a) Einführung der genannten wäßrigen Lösung in eine erste Filtrationsstufe einer zweistufigen Kaskaden-Tangential-Filtrations-Apparatur, die Filtrationsmembranen aufweist, die eine Zurückweisung (Rejektion) von Natriumchlorid von nicht höher als 85 % und eine Zurückweisung (Rejektion) von Raffinose von nicht höher als 85 % aufweisen;
   b) Überführung des Filtrats/Permeats aus der ersten Stufe in die zweite Filtrationsstufe, wobei das Verhältnis zwischen den Filtrationsmembran-Oberflächen zwischen der ersten und der zweiten Stufe in dem Bereich von 1,5 bis 6 liegt;
   c) kontinuierliches Recyclisieren des Retentats aus der zweiten Filtrationsstufe in das Retentat aus der ersten Filtrationsstufe, Verdünnen des resultierenden Retentats mit Wasser, wobei die Menge des Wassers 12 kg pro mol der organischen Verbindung, die gereinigt werden soll, nicht übersteigt;
   d) Wiederholung der Stufen (b) bis (c) mit dem resultierenden Retentat zur Erzielung eines solchen Grades der Reinigung der genannten organischen Verbindung in dem Schluß-Retentat aus der ersten Stufe, daß die Gesamtmenge der restlichen Verunreinigungen 10 % des Anfangswertes nicht übersteigt.

2. Verfahren nach Anspruch 1, dadurch gekennzeichnet, daß die Stufe (d) wiederholt wird, bis die Gesamtmenge der restlichen Verunreinigungen 5 % des Anfangswertes nicht übersteigt.

3. Verfahren nach den Ansprüchen 1 bis 2, dadurch gekennzeichnet, daß das Verhältnis der Filtrationsmembran-Oberflächen zwischen der ersten Stufe und der zweiten Stufe in dem Bereich von 2,5 bis 4 liegt.

4. Verfahren nach den Ansprüchen 1 bis 3, dadurch gekennzeichnet, daß die Membranen eine Zurückweisung (Rejektion) von Natriumchlorid von nicht höher als 70 % und eine Zurückweisung (Rejektion) von Raffinose von höher als 95 % aufweisen.

5. Verfahren nach einem der vorhergehenden Ansprüche, worin die Arbeitstemperatur der ersten Stufe und der zweiten Stufe in dem Bereich von 10 bis 90°C liegt.

6. Verfahren nach einem der vorhergehenden Ansprüche, worin die Arbeitstemperatur der ersten Stufe und der zweiten Stufe in dem Bereich von 25 bis 45°C liegt.

7. Verfahren nach einem der vorhergehenden Ansprüche, worin der Arbeitsdruck der ersten Stufe und der zweiten Stufe in dem Bereich von 1,5 bis 5,5 MPa liegt.

8. Verfahren nach einem der vorhergehenden Ansprüche, worin der Arbeitsdruck der ersten Stufe und der zweiten Stufe in dem Bereich von 2 bis 3,5 MPa liegt.

9. Verfahren nach Anspruch 1, wobei die Filtrations-Apparatur mit einer Verdampfungs-Kondensator-Gruppe verbunden ist zur Rückgewinnung der flüchtigen Komponenten aus dem Permeat der zweiten Stufe.

10. Verfahren nach den Ansprüchen 1 bis 9, worin das Schluß-Retentat über eine Sicherheitseinrichtung von anionischen und kationischen Ionenaustauscherharzen laufen gelassen wird.

11. Verfahren nach Anspruch 1, worin in der Stufe (c) die Wassermenge einen Wert von 8 kg pro mol der organischen Verbindung nicht übersteigt.

12. Verfahren nach Anspruch 1, worin die nicht-ionische, wasserlösliche, jodierte kontrastverbessernde organische Verbindung das Röntgen-Opazifierungsmittel (L)-5-(2-Hydroxy-propionyl)amino-2,4,6-trijodobis-(1,3-dihydroxy-isopropyl)isophthalamid ist.

13. Verfahren nach Anspruch 1, worin die nicht-ionische, wasserlösliche, jodierte kontrastverbessernde organische Verbindung das Röntgen-Opazifierungsmittel 5-(N-Methyl-hydroxyacetyl)amino-2,4,6-trijodobis-(2,3-dihydroxypropyl)-isophthalamid ist.

14. Verfahren nach Anspruch 1, worin die nicht-ionische, wasserlösliche, jodierte kontrastverbessernde organische Verbindung das Röntgen-Opazifierungsmittel 1,3-Bis-[3-(L-2-Hydroxy-propionyl)amino-5-(1,3-dihydroxy-isopropyl)aminocarbonyl-2,4,6-trijodo-benzoyl-amino]-2-hydroxypropan ist.

15. Zwei-Stufen-Tangentialfiltrations-Anlage zur Durchführung des Verfahrens nach den Ansprüchen 1 bis 14, die umfaßt zwei Filtrationsstufen (A) und (B), die in Reihe miteinander verbunden sind, wobei das Verhältnis der Filtrationsmembran-Oberflächen zwischen der ersten Stufe (A) und der zweiten Stufe (B) in dem Bereich von 1,5 bis 6 liegt, und einen Einlaß zur Einführung einer rohen Lösung der jodierten organischen Verbindung in die genannte erste Stufe (A), dadurch gekennzeichnet, daß
   - der Retentat-Auslaß aus der genannten ersten Stufe (A) mittels eines Recyclisierungskreislaufs (e) mit der gleichen ersten Stufe (A) verbunden ist;
   - der Permeat-Auslaß der ersten Stufe (A) mittels des Kreislaufs (c) mit dem Beschickungs-Einlaß der zweiten Stufe verbunden ist;
   - der Retentat-Auslaß der zweiten Stufe (B) mittels eines Recyclisierungskreislaufs (f) mit der ersten Stufe (A) verbunden ist;
   - die ersten Stufe (A) mit einer Verbindung für die Auffüllungs-Wasserleitung (b) versehen ist;
   - der Permeat-Auslaß der zweiten Stufe (B) zur Abfallbeseitigung oder durch den Kreislauf (d) in ein sekundäres Rückgewinnungssystem geht;
   - der Schluß-Retentat-Auslaß der ersten Stufe (A) in den Haupt-Rückgewinnungs-Kreislauf (g) geht zur Rückgewinnung (Abtrennung) der reinen jodierten Verbindung.

16. Filtrationsanlage nach Anspruch 15, worin der Permeat-Auslaß der zweiten Stufe (B) durch den Kreislauf (d) mit einer Verdampfungs-Einheit (C) verbunden ist, die mit einer Kondensatoreinheit ausgestattet ist.

17. Filtrationsanlage nach Anspruch 15, worin der Schluß-Retentat-Auslaß der ersten Stufe (A) über den Kreislauf (g) mit einer Sicherheitseinrichtung von anionischen und kationischen Harzen verbunden ist.

**Revendications**

1. Un procédé pour la concentration et la purification de composés organiques iodés, non ioniques, hydrosolubles, renforçateurs de contraste, à partir de leurs solutions aqueuses, dans lequel leur concentration se situe entre 15 et 60 % en poids et qui contiennent, comme impuretés dissoutes, des sels minéraux et/ou des corps réagissants organiques résiduels hydrosolubles non consommés de masse relative inférieure à environ 200 et/ou des solvants organiques hydrosolubles, ledit procédé comprenant les étapes suivantes :

EP 0 575 360 B1

a) introduire ladite solution aqueuse dans un premier étage de filtration d'un appareil de filtration tangentielle à deux étages reliés en série, ledit appareil comportant des membranes filtrantes, lesdites membranes ayant un taux de rejet du chlorure de sodium non supérieur à 85 % et un taux de rejet du raffinose supérieur à 85 % ;

b) faire passer le filtrat/perméat dudit premier étage audit second étage de filtration, le rapport des surfaces des membranes filtrantes entre lesdits premier et second étages étant compris entre 1,5 et 6 ;

c) recycler en continu le rétentat dudit second étage de filtration dans le rétentat dudit premier étage de filtration, diluer le rétentat résultant avec de l'eau, la quantité de cette eau ne dépassant pas 12 kg par mole dudit composé organique soumis à la purification ;

d) répéter b) à c) sur ledit rétentat résultant pour obtenir un degré de purification dudit composé organique dans le rétentat final provenant dudit premier étage tel que la quantité totale d'impuretés résiduelles ne dépasse pas 10 % de la quantité initiale.

2. Le procédé selon la revendication 1, caractérisé en ce que l'étape d) est répétée pour atteindre une quantité totale d'impuretés résiduelles ne dépassant pas 5 % de la quantité initiale.

3. Un procédé selon les revendications 1 et 2, caractérisé en ce que le rapport desdites surfaces des membranes filtrantes entre lesdits premier et second étages est compris entre 2,5 et 4.

4. Le procédé selon les revendications 1 à 3, caractérisé en ce que lesdites membranes ont un taux de rejet du chlorure de sodium non supérieur à 70 % et un taux de rejet du raffinose supérieur à 95 %.

5. Un procédé selon l'une quelconque des revendications précédentes, dans lequel la température de travail desdits premier et second étages se situe entre 10° et 90°C.

6. Un procédé selon l'une quelconque des revendications précédentes, dans lequel la température de travail desdits premier et second étages se situe entre 25° et 45°C.

7. Un procédé selon l'une quelconque des revendications précédentes, dans lequel la pression de travail desdits premier et second étages se situe entre 1,5 et 5,5 MPa.

8. Un procédé selon l'une quelconque des revendications précédentes, dans lequel la pression de travail desdits premier et second étages se situe entre 2 et 3,5 MPa.

9. Un procédé selon la revendication 1, dans lequel ledit appareil de filtration est connecté à un groupe d'évaporation-condensation pour recueillir les composants volatils en provenance du perméat dudit second étage.

10. Un procédé selon les revendications 1 à 9, dans lequel ledit rétentat final est amené à traverser un dispositif de garde comprenant des résines échangeuses d'ions anionique et cationique.

11. Un procédé selon la revendication 1, dans lequel, dans l'étape c), ladite quantité d'eau ne dépasse pas 8 kg par mole dudit composé organique.

12. Un procédé selon la revendication 1, dans lequel le composé organique iodé, non ionique, hydrosoluble, renforçateur de contraste, est l'agent opacifiant radiologique (L)-5-(2-hydroxy-propionyl)amino-2,4,6-triiodo-bis(1,3-dihydroxy-isopropyl)isophtalamide.

13. Un procédé selon la revendication 1, dans lequel le composé organique iodé, non ionique, hydrosoluble, renforçateur de contraste, est l'agent opacifiant radiologique5-(N-méthyl-hydroxyacétyl)amino-2,4,6-triiodo-bis(2,3-dihydroxy-propyl)isophtalamide.

14. Un procédé selon la revendication 1, dans lequel le composé organique iodé, non ionique, hydrosoluble, renforçateur de contraste, est l'agent opacifiant radiologique1,3-bis[3-(L-2-hydroxy-propionyl)amino-5-(1,3-dihydroxy-isopropyl)aminocarbonyl-2,4,6-triiodo-benzoylamino]-2-hydroxypropane.

15. Une installation de filtration tangentielle à deux étages pour la mise en oeuvre du procédé des revendications 1 à 14, ladite installation comprenant deux étages de filtration (A) et (B) reliés en série, le rapport des surfaces des membranes filtrantes entre ledit premier étage (A) et ledit second étage (B) étant compris entre 1,5 et 6, une entrée pour l'alimentation dudit premier étage (A) avec une solution brute de composé organique iodé, caractérisée en ce que :

- la sortie de <u>rétentat</u> dudit premier étage (A) est reliée au moyen d'un circuit de recyclage e) au même premier étage (A) ;
- la sortie de <u>perméat</u> dudit premier étage (A) est reliée à l'entrée d'alimentation dudit second étage (B) au moyen du circuit c) ;
- la sortie de <u>rétentat</u> dudit second étage (B) est reliée au moyen d'un circuit de recyclage f) audit premier étage (A) ;
- ledit premier étage (A) est pourvu d'un raccord pour la conduite d'eau d'appoint b) ;
- la sortie de <u>perméat</u> dudit second étage (B) va à l'égout ou à un système de récupération secondaire au moyen du circuit d) ;
- la sortie de <u>rétentat</u> final dudit premier étage (A) va au circuit de récupération principal g) pour la récupération du composé iodé pur.

16. Une installation de filtration selon la revendication 15, dans laquelle la sortie de <u>perméat</u> dudit second étage (B) est reliée par le circuit d) à une unité d'évaporation (C), ladite unité d'évaporation étant équipée d'une unité de condensation.

17. Une installation de filtration selon la revendication 15, dans laquelle la sortie de <u>rétentat</u> final dudit premier étage (A) est reliée par le circuit g) à un dispositif de garde comprenant des résines anionique et cationique.

Figure 1 - Filtration plant scheme

| | | |
|---|---|---|
| A | = | Filtrating units 1st stage |
| B | = | Filtrating unit 2nd stage |
| C | = | Evaporating unit |
| D | = | Cationic and anionic resin column |
| a) | = | Raw solution of iodinated contrast medium |
| b) | = | Make-up water |
| c) | = | First permeate (to the second stage) |
| d) | = | Final permeate (to the evaporator) |
| e) | = | First stage/unit retentate |
| f) | = | Second stage/unit retentate |
| g) | = | Final retentate (containing the purified contrast medium) |
| h) | = | Eluate (to the recovery of the contrast medium) |
| i) | = | Residue (to the recovery of valuable compounds) |
| l) | = | Reaction solvent (to the recovery) |
| m) | = | Water |